(19) Europäisches Patentamt
European Patent Office
Office européen des brevets

(11) Veröffentlichungsnummer: **0 086 477**
**A1**

(12) # EUROPÄISCHE PATENTANMELDUNG

(21) Anmeldenummer: **83101347.9**

(22) Anmeldetag: **12.02.83**

(51) Int. Cl.³: **C 07 D 285/06**, A 01 N 43/82

(30) Priorität: **17.02.82 DE 3205639**

(43) Veröffentlichungstag der Anmeldung: **24.08.83**
**Patentblatt 83/34**

(84) Benannte Vertragsstaaten: **AT BE CH DE FR GB IT LI NL**

(71) Anmelder: **HOECHST AKTIENGESELLSCHAFT,**
**Postfach 80 03 20, D-6230 Frankfurt am Main 80 (DE)**

(72) Erfinder: **Kübel, Börries, Dr., Kuckucksweg 14,**
**D-6233 Kelkheim (Taunus) (DE)**
Erfinder: **Koch, Manfred, Dr., Kreuzheck 2,**
**D-6239 Eppstein/Taunus (DE)**
Erfinder: **Bauer, Klaus, Dr., Kolpingstrasse 7,**
**D-6054 Rodgau (DE)**
Erfinder: **Bieringer, Hermann, Dr., Eichenweg 26,**
**D-6239 Eppstein/Taunus (DE)**

(54) **Neue Chloracetanilide, Verfahren zu ihrer Herstellung, ihre Verwendung als Herbizide und sie enthaltende Mittel.**

(57) Chloracetanilide der Formel

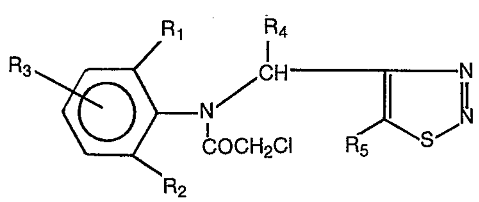

(I)

worin
$R_1$ Alkyl, $R_2$ Wasserstoff, Alkyl, Alkenyl, Alkoxymethyl, Halogen oder Alkoxy, $R_3$ Wasserstoff, Chlor, Brom oder Methyl, $R_4$ Wasserstoff, Alkyl oder Alkoxycarbonyl, $R_5$ Wasserstoff, Alkyl, Halogen, Carboxy oder Alkoxycarbonyl oder $R_4$ und $R_5$ zusammen eine Alkylengruppe bedeuten, und deren Salze sind wirksame (Selektiv)herbizide gegen mono- und dikotyle Schadpflanzen im Vor- und Nachauflauf.

EP 0 086 477 A1

0086477

HOECHST AKTIENGESELLSCHAFT HOE 82/F 027 Dr.TG/Wa

Neue Chloracetanilide, Verfahren zu ihrer Herstellung, ihre Verwendung als Herbizide und sie enthaltende Mittel

Gegenstand der vorliegenden Anmeldung sind neue Chloracetanilide der allgemeinen Formel

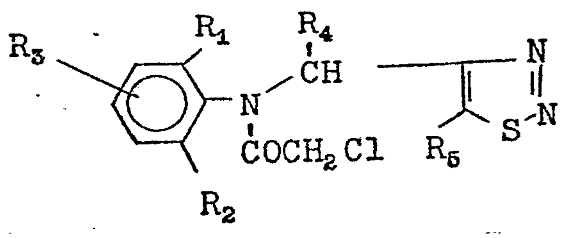

(I)

worin

$R_1$ $(C_1-C_3)$Alkyl,

$R_2$ Wasserstoff, $(C_1-C_3)$Alkyl, $(C_3-C_5)$Alkenyl, $(C_1-C_3)$Alkoxymethyl, Halogen oder $(C_1-C_3)$Alkoxy

$R_3$ Wasserstoff, Chlor, Brom oder Methyl,

$R_4$ Wasserstoff, $(C_1-C_3)$Alkyl oder COO-$(C_1-C_4)$Alkyl

$R_5$ Wasserstoff, $(C_1-C_3)$Alkyl, Halogen, Carboxy oder $(C_1-C_4)$Alkoxycarbonyl

oder

$R_4$ und
$R_5$ zusammen Trimethylen oder Ethylen.

bedeuten, sowie, falls $R_5$= COOH, deren Salze mit Basen.

BAD ORIGINAL

Besonders bevorzugt sind solche Verbindungen der Formel I, worin $R_4$ und $R_5$ Wasserstoff bedeuten.

Die Chloracetanilide der Formel I werden hergestellt, indem man die ebenfalls neuen N-substituierten Aniline der Formel II

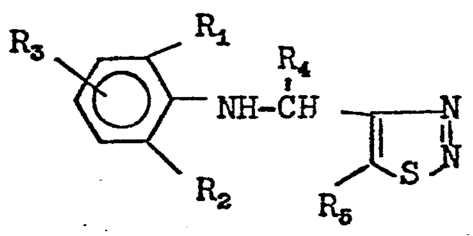 (II)

mit Chloracetylierungsmitteln behandelt.

Die Umsetzung kann in An- oder Abwesenheit von gegenüber den Reaktionsteilnehmern inerten Lösungs- oder Verdünnungsmitteln durchgeführt werden. Es kommen beispielsweise in Frage: aliphatische, aromatische oder halogenierte Kohlenwasserstoffe wie Benzol, Toluol, Xylole, Petroläther, Chlorbenzol, Methylenchlorid, Aethylenchlorid, Chloroform; Ether wie Di-alkylether, Dioxan, Tetrahydrofuran; Nitrile wie Acetonitril, N,N-dialkylierte Amide wie Dimethylformamid; ferner Dimethylsulfoxid sowie Gemische dieser Lösungsmittel untereinander. Die Umsetzungstemperaturen liegen zwischen 0 °C und dem Siedepunkt des verwendeten Lösungsmittels, vorzugsweise zwischen 20 und 140 °C.

Geeignete Chloracetylierungsmittel sind Chloressigsäurehalogenide wie Chloressigsäurechlorid, Chloressigsäurebromid, Chloressigsäureanhydrid, Chloressig-

BAD ORIGINAL

säure und Chloressigsäureester. Die Umsetzung kann, insbesondere bei Verwendung von Chloressigsäurehalogeniden, in An- oder Abwesenheit von säurebindenden Mitteln durchgeführt werden.
Als solche kommen in Betracht:
tertiäre Amine, wie z.B. Triethylamin und Pyridin, anorganische Basen wie Oxide, Hydroxide, Carbonate bzw. Hydrogencarbonate. Als säurebindendes Mittel kann ferner im Ueberschuß eingesetztes Anilin der Formel II dienen.

Die Herstellung von Verbindungen der Formel II erfolgt durch Umsetzung von 4-Halogenmethyl-1.2.3-thiadiazolen der Formel III mit Anilinen der Formel IV in Gegenwart von säurebindenden Mitteln:

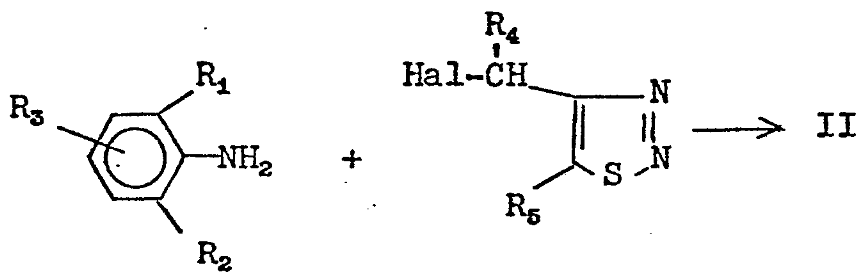

IV III

Hal bedeutet Halogen, bevorzugt Chlor und Brom.

Die Umsetzung wird in gegenüber den Reaktionsteilnehmern inerten Lösungs- oder Verdünnungsmitteln oder aber in überschüssigem Anilin der Formel IV durchgeführt. Als Lösungsmittel kommen in Frage: Aliphatische,

BAD ORIGINAL

aromatische oder halogenierte Kohlenwasserstoffe wie Benzol, Toluol, Xylole, Petrolether, Chlorbenzol, Methylenchlorid, Ethylenchlorid, Chloroform; Ether wie Dialkylether, Dioxan, Tetrahydrofuran; Nitrile wie Acetonitril, N,N-dialkylierte Amide wie Dimethylformamid; ferner Dimethylsulfoxid sowie Gemische dieser Lösungsmittel untereinander. Die Umsetzungstemperaturen liegen zwischen 60 °C und dem Siedepunkt des verwendeten Lösungsmittels, vorzugsweise zwischen 80 und 140 °C.

Als säurebindende Mittel kommen tertiäre Amine, wie z.B. Triethylamin, Pyridin oder anorganische Basen wie Oxide oder Hydroxide und Carbonate bzw. Hydrogencarbonate von Alkali- und Erdalkalimetallen in Frage. Als säurebindendes Mittel kann aber auch im Ueberschuß eingesetztes Anilin der Formel IV dienen.

Soweit gewünscht, kann in den Verbindungen der Formel II anschließend der Rest $R_5$ abgewandelt werden, z.B. durch Verseifung von $R_5$= COO Alkyl, Veresterung, Umesterung oder Decarboxylierung.

Die zur Herstellung der Verbindungen II benötigten Aniline IV sind allgemein bekannte Verbindungen der organischen Chemie. Als Beispiele seien genannt: 2-Methylanilin, 2-Isopropylanilin, 2,6-Dimethylanilin, 2-Methyl-6-ethylanilin, 2,6-Diethylanilin, 2-Ethyl-6-isopropylanilin, 2-Methyl-6-isopropylanilin, 2,6-Diisopropylanilin, 2-Chlor-6-methylanilin, 2.3.6-Trimethylanilin, 6-Ethyl-2.3-dimethylanilin, 3-Chlor-2.6-dimethylanilin, 2,4-Dichlor-6-methylanilin.

BAD ORIGINAL

Die 4-Halogenmethyl-1.2.3-thiadiazole III sind - mit einigen Ausnahmen (vgl. J. Heterocycl. Chem 13, 301 (1976); 17, 1639 (1980)) neu. Sie können entweder, wie die genannten Verbindungen, durch Bromierung entsprechender 4-Alkyl-1.2.3-thiadiazole mit N-Bromsuccinimid oder vorzugsweise durch Umsetzung entsprechender Hydrazone der Formel V

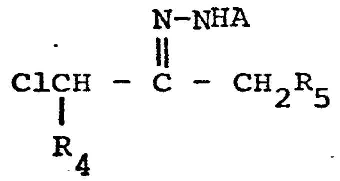

V

mit Thionylchlorid entsprechend J. Am. Chem. Soc. 77, 5359-5364 (1955) hergestellt werden.

Die Hydrazone V sind ihrerseits aus entsprechenden Chlorketonen VI und Hydrazinen VII erhältlich. Vorteilhaft ist bei diesem Verfahren, daß man von billigen Chlorketonen ausgehen kann und sich so die relativ teure Bromierung erspart. Dabei ist es überraschend, daß die Hydrazine VII unter geeigneten Bedingungen nur mit der Ketogruppe von VI und nicht mit dem Chloratom reagieren.

Schema 2

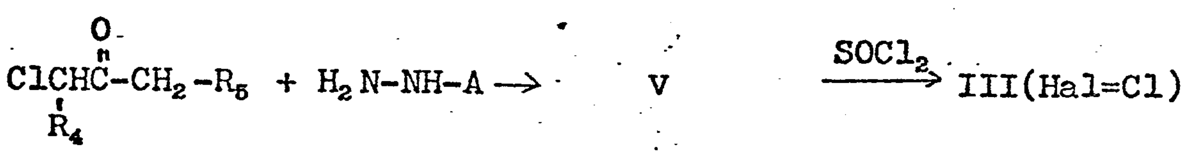


VI VII

Dabei bedeutet

A $CONH_2$, $SO_2-C_6H_4-CH_3$ oder $COO$ Alkyl, vorzugsweise $COOCH_3$.

BAD ORIGINAL

Die Umsetzung von VI mit VII erfolgt in niederen Alkoholen wie Methanol oder Ethanol und unter Zusatz mindestens äquimolarer Mengen einer Säure, beispielsweise Essigsäure, Ameisensäure oder Propionsäure. Die Reaktionszeit beträgt 1 bis 20 h und die Temperatur 0 bis 80°C.

Aufgrund der gehinderten Rotation um die N-C(Phenyl)-Achse können bei $R_1 \neq R_2$ Enantiomere auftreten. Für $R_4 \neq H$ treten im Falle von $R_1 = R_2$ Enantiomere, im Falle von $R_1 \neq R_2$ zwei Diastereomerenpaare auf. Die Erfindung betrifft sowohl die reinen Enantiomere und Diastereomere(npaare) als auch deren Gemische.

Die Verbindungen mit der allgemeinen Formel I besitzen eine hervorragende herbizide Wirkung gegen annuelle und perennierende Schadpflanzen im Vor- und Nachauflauf. Je nach Dosis kommt es zu einer Hemmung des Pflanzenwachstums oder zu einer vollständigen Abtötung der Pflanzen.

Unter den bekämpfbaren Arten befinden sich wirtschaftlich wichtige Schadgräser wie Setaria (Borstenhirse), Digitaria (Fingerhirse), Echinochloa (Hühnerhirse), Alopecurus (Ackerfuchsschwanz), Avena (Flughafer), Cyperaceen (Sauergräser) und Agropyron (Quecke) sowie auch dikotyle Unkräuter wie Matricaria (Kamille), Chrysanthemum (Saatwucherblume) und Amaranthus (Amaranth). Daneben schonen die neuen Verbindungen in den herbizid wirksamen Aufwandmengen viele Kulturpflanzenarten, so daß sie in wichtigen Großkulturen zur selektiven Bekämpfung von grasartigen und breitblättrigen Unkräutern eingesetzt werden können.

BAD ORIGINAL

So kann man mit den erfindungsgemäßen Mitteln beispielsweise Hühnerhirse und Amaranth in Mais, Fingerhirse und Borstenhirse in Soja und Baumwollkulturen wirksam bekämpfen, ohne daß ein Kulturschaden zu beobachten ist. Aber auch in Reis lassen sich die neuen erfindungsgemäßen Verbindungen mit Erfolg zur Bekämpfung wichtiger Schadpflanzen wie z.B. Hühnerhirse und Cyperaceen-Arten einsetzen.

Darüberhinaus können sie auch in breitblättrigen Kulturen der gemäßigten Breiten z.B. in Raps, selektiv zur Kontrolle wirtschaftlich wichtiger Schadpflanzen wie beispielsweise Ackerfuchsschwanz und Ausfallgetreide verwendet werden.

Hinsichtlich Selektivität und Wirksamkeit sind die neuen Verbindungen bekannten Pflanzenschutzmitteln wie 2,6-Dimethyl-N-methoxymethyl-chloracetanilid (Alachlor) und 2-Methyl-6-ethyl-N-methoxymethyl-chloracetanilid (Metolachlor) bei einer Reihe von Pflanzen überlegen.

Die erfindungsgemäßen Mittel enthalten die Wirkstoffe der Formel I im allgemeinen zu 2 bis 95 Gew.-%. Sie können als Spritzpulver, emulgierbare Konzentrate, versprühbare Lösungen, Stäubemittel oder Granulate in den üblichen Zubereitungen angewendet werden.

Spritzpulver sind in Wasser gleichmäßig dispergierbare Präparate, die neben dem Wirkstoff außer einem Verdünnungsmittel oder Inertstoff noch Netzmittel, z.B. polyoxethylierte Alkylphenole, polyoxethylierte Fettalkohole, Alkyl- oder Alkylphenyl-sulfonate und Dispergiermittel, z.B. ligninsulfonsaures Natrium, 2,2'-dinaphthylmethan-6,6'-disulfonsaures Natrium, dibutylnaphthalinsulfonsaures Natrium oder auch oleylmethyltaurinsaures Natrium, enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden:

Alkylarylsulfonsaure Calciumsalze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyether, Sorbitanfettsäureester, Polyoxethylensorbitan-fettsäureester oder Polyoxethylensorbitester.

Stäubemittel erhält man durch Vermahlen des Wirkstoffes mit fein verteilten, festen Stoffen, z.B. Talkum, natürlichen Tonen, wie Kaolin, Bentonit, Pyrophillit oder Diatomeenerde.

BAD ORIGINAL

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol,polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranalien üblichen Weise - gewünschtenfalls in Mischung mit Düngemitteln - hergestellt werden.

Bei herbiziden Mitteln können die Konzentrationen der Wirkstoffe in den handelsüblichen Formulierungen verschieden sein.

In Spritzpulvern variiert die Wirkstoffkonzentration z.B. zwischen etwa 10 % und 80 %, der Rest besteht aus den oben angegebenen Formulierungszusätzen. Bei emuligierbaren Konzentraten kann die Wirkstoffkonzentration gleichfalls etwa 10 % bis 80 % betragen. Stäubeförmige Formulierungen enthalten meistens 5 bis 20 % an Wirkstoff, versprühbare Lösungen etwa 2 bis 20 %. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

BAD ORIGINAL

Zur Anwendung werden die handelsüblichen Konzentrate gegebenenfalls in üblicher Weise verdünnt, z.B. bei Spritzpulvern und emulgierbaren Konzentraten mittels Wasser. Stäubeförmige und granulierte Zubereitungen sowie versprühbare Lösungen werden vor der Anwendung nicht mehr mit weiteren inerten Stoffen verdünnt. Mit den äußeren Bedingungen wie Temperatur, Feuchtigkeit u.a., variiert die erforderliche Aufwandmenge. Sie beträgt im allgemeinen zwischen 0,1 und 10 kg/ha, vorzugsweise etwa 0,1 bis 5,0 kg/ha Wirkstoff. Der erfindungsgemäße Wirkstoff kann mit anderen Herbiziden, Insektiziden und Fungiziden kombiniert werden.

BAD ORIGINAL

## Herstellungsbeispiele

### A. 4-Halogenmethyl-1.2.3-thiadiazole III

#### Beispiel 1

4-Chlormethyl-1.2.3-thiadiazol-5-carbonsäuremethylester

Man löst 138,8 g (1 mol) γ-Chloracetessigsäuremethylester in 1 Liter Methanol und 60 ml (1 mol) Eisessig. Dazu fügt man 90 g (1 mol) Hydrazinoameisensäuremethylester, rührt 1 h unter Rückfluß und läßt über Nacht bei Raumtemperatur stehen. Dann dampft man i. V. ein und kristallisiert den Rückstand aus Ether um. Ausbeute 164,8 g (74 %) γ-Chloracetessigsäuremethylester-methoxycarbonylhydrazon; gelbe Kristalle mit Schmp. 85 - 87 °C.
155,9 g (0,7 mol) dieser Kristalle fügt man portionsweise unter Rühren bei 15 - 20 °C während 1 h zu 400 ml Thionylchlorid. Man rührt noch 2 h bei Raumtemperatur, läßt über Nacht stehen und zieht dann das überschüssige Thionylchlorid i. V. ab. Den Rückstand destilliert man an der Oelpumpe. Ausbeute 94,3 g (70 %), Sdp. 105 - 110 °C/2 mbar; Schmp. 49 - 52 °C.

#### Beispiel 2

5-Chlor-4-chlormethyl-1.2.3-thiadiazol

Man versetzt eine Lösung von 42,5 g (0,335 mol) 1.3-Dichloraceton in 50 ml Methanol bei 20 °C unter Rühren mit einer Lösung von 30 g (0,334 mol) Hydrazinoameisen-

BAD ORIGINAL

säuremethylester und 21 ml Eisessig in 150 ml Methanol, rührt 0,5 h und läßt noch 1,5 h stehen. Dann wird abgesaugt und mit wenig Methanol gewaschen. Ausbeute 50,6 g (78 %) 1.3-Dichloraceton-methoxycarbonyl-hydrazon, weiße Kristalle vom Schmp. 136°C.
Diese werden bei ca. 15°C portionsweise unter Rühren in 120 ml Thionylchlorid eingetragen. Man rührt noch 3 h, läßt über Nacht stehen und zieht dann im Vakuum den Thionylchloridüberschuß ab. Der Rückstand wird in Ether gelöst, mit Wasser und Bicarbonatlösung neutral gewaschen, getrocknet und eingedampft. Ausbeute 36,8 g (92 %); hellbraune Flüssigkeit, die langsam kristallisiert.
Schmp.: 38°C.

Beispiel 3

2-Chlor-2-(1.2.3-thiadiazol-4-yl)-essigsäuremethylester

45,5 g (0,3 mol) α-Chloracetessigsäure-methylester, 20 ml Eisessig und 27 g (0,3 mol) Hydrazinoameisensäuremethylester werden in 200 ml Methanol gelöst und über Nacht bei Raumtemperatur stehengelassen. Man dampft im Vakuum ein, nimmt den Rückstand in Dichlormethan auf, wäscht mit Bicarbonatlösung, trocknet und tropft die erhaltene Lösung bei 15-20°C unter Rühren zu 80 ml Thionylchlorid. Nach 6 stündigem Rühren und Stehen über Nacht dampft man im Vakuum ein und extrahiert den öligen Rückstand mit Benzin. Einfampfen des Extraktes liefert das Produkt mit 29,9 g (52 %) Ausbeute als Öl von 86%iger Reinheit.

BAD ORIGINAL

In analoger Weise können folgende Verbindungen III hergestellt werden:

| Nr. | Hal | $R_4$ | $R_5$ |
|---|---|---|---|
| 4 | Cl | H | $COOC_4H_9$-(i) |
| 5 | Br | $C_2H_5$ | $COOC_2H_5$ |
| 6 | Cl | $COOC_2H_5$ | $C_2H_5$ |

B. Anilinomethyl-1.2.3-thiadiazole der Formel II

Beispiel 7

4-(2.6-Dimethylphenylaminomethyl)-1.2.3-thiadiazol-5-carbonsäure-methylester

Eine Mischung aus 20 g (0,104 mol) 4-Chlormethyl-1.2.3-thiadiazol-5-carbonsäuremethylester (Beispiel 1) und 50,3 g 2.6-Dimethylanilin wird 50 h bei 90°C gerührt. Anschließend versetzt man das Gemisch mit 150 ml Toluol und 60 ml 2 N Natronlauge, rührt kräftig durch und trennt anschließend die organische Phase ab. Eindampfen, Abdestillieren des Dimethylanilins im Hochvakuum und Kristallisieren aus Cyclohexan ergibt 19,1 g (66 %); Schmp.: 94°C.

Beispiel 8

4-(2.6-Dimethylphenylaminomethyl)-1.2.3-thiadiazol

a) Man rührt 13 g (68 mmol) 4-Chlormethyl-1.2.3-thiadiazol-5-carbonsäure-methylester (Beispiel 1) und

30 g (248 mmol) 2.6-Dimethylanilin 7 h bei 90°C, fügt anschließend 100 ml Ethanol, 10 ml Wasser und 10 g Kaliumhydroxid zu, rührt weitere 36 h bei Raumtemperatur und gießt dann auf 200 ml Wasser. Man schüttelt dreimal mit Ether aus und säuert auf pH 2 an. Es scheidet sich ein Öl ab, das durch Anreiben zur Kristallisation gebracht wird. Man saugt ab, wäscht mit Wasser und mit etwas Methanol und trocknet im V. Ausbeute 14,3 g (80 %) 4(2.6-Dimethylphenylaminomethyl)-1.2.3-thiadiazol-5-carbonsäure, Schmp.: 157°C (Zers.).

b) 13,2 g (50 mmol) der Verbindung aus a) werden 15 min. bei 165°C gerührt, wonach die Kohlendioxidabspaltung beendet ist. Ausbeute 10,95 g (quantitativ); hellbraunes Öl.
$^1$H-NMR : $\delta$ = 2.25 (s; 6 H); 3.35 (s; 1 H), 4.65 (s; 2 H), 6.8 - 7.2 (m; 3 H), 8.2 (s; 1 H)

Analog zu den Beispielen 7 und 8 können u. a. folgende Verbindungen der Formel II erhalten werden:

| Nr. | $R_1$ | $R_2$ | $R_3$ | $R_4$ | $R_5$ | Schm.(°C) |
|---|---|---|---|---|---|---|
| 9 | $CH_3$ | $CH_3$ | H | H | Cl | Oel |
| 10 | $CH_3$ | $CH_3$ | H | $COOCH_3$ | H | Oel |
| 11 | $CH_3$ | $CH_2-CH=CH_2$ | H | H | $COOCH_2CH(CH_3)_2$ | |
| 12 | $CH_3$ | $CH_3$ | $3-CH_3$ | H | $COOCH_3$ | |
| 13 | $CH_3$ | $CH_3$ | $3-CH_3$ | H | COOH | |
| 14 | $CH_3$ | $CH_3$ | $3-CH_3$ | H | H | |
| 15 | $CH_3$ | $C_2H_5$ | H | H | $COOCH_3$ | 80 |
| 16 | $CH_3$ | $C_2H_5$ | H | H | Cl | |
| 17 | $CH_3$ | $C_2H_5$ | H | H | COOH | 134°C(Zers.) |
| 18 | $CH_3$ | $C_2H_5$ | H | H | H | Oel |
| 19 | $CH_3$ | $CH_3$ | 3-Cl | H | H | |
| 20 | $C_2H_5$ | $C_2H_5$ | H | H | $COOCH_3$ | 69 |
| 21 | $C_2H_5$ | $C_2H_5$ | H | H | COOH | 120(Zers.) |

BAD ORIGINAL

| Nr. | $R_1$ | $R_2$ | $R_3$ | $R_4$ | $R_5$ | Schmp. (°C) |
|---|---|---|---|---|---|---|
| 22 | $CH_3$ | $C_2H_5$ | H | $C_2H_5$ | $COOC_2H_5$ | |
| 23 | $C_2H_5$ | $C_2H_5$ | H | H | H | Oel |
| 24 | $CH_3$ | $C_2H_5$ | H | $COOCH_3$ | H | |
| 25 | $CH_3$ | Cl | 4-Cl | H | COOH | 124 |
| 26 | $CH_3$ | Cl | 4-Cl | H | H | Oel |
| 27 | $CH_3$ | $CH_3$ | H | $C_2H_5$ | $COOCH_3$ | |
| 28 | $CH_3$ | $CH_3$ | H | $COOC_2H_5$ | $C_2H_5$ | |
| 29 | $CH_3$ | H | H | H | H | |
| 30 | $CH_3$ | $CH_3$ | H | $C_2H_5$ | H | |
| 31 | $i-C_3H_7$ | H | H | H | H | |
| 32 | $i-C_3H_7$ | $i-C_3H_7$ | H | H | H | |

C. Chloressigsäure-N-(1.2.3-thiadiazol-4-ylmethyl)-anilide der Formel I

Beispiel 33

Chloressigsäure-N-(1.2.3-thiadiazol-4-ylmethyl)-2.6-dimethylanilid

10.95 g (0.05 mol) der Verbindung aus Beispiel 8 b) in 100 ml Toluol gelöst. Bei ca. 40 °C läßt man unter Rühren 3.95 ml (0.052 mol) Chloracetylchlorid zutropfen, erwärmt dann innerhalb 30 min zum Rückfluß und rührt noch 1,5 h bei dieser Temperatur. Nach dem Abkühlen dampft man i. V. ein und kristallisiert aus Methanol um. Ausbeute 9,5 g (64 %), Schmp.: 94°C.

/

BAD ORIGINAL

Analog zu Beispiel 33 können die folgenden Verbindungen I erhalten werden

| Nr. | $R_1$ | $R_2$ | $R_3$ | $R_4$ | $R_5$ | Schmp. (°C) |
|---|---|---|---|---|---|---|
| 34 | $CH_3$ | $CH_3$ | H | H | $COOCH_3$ | 112 |
| 35 | $CH_3$ | $CH_3$ | H | H | Cl | 83 |
| 36 | $CH_3$ | $CH_3$ | 3-$CH_3$ | H | H | |
| 37 | $CH_3$ | $CH_3$ | 3-$CH_3$ | H | COOH | |
| 38 | $CH_3$ | $CH_3$ | H | $C_2H_5$ | $COOCH_3$ | |
| 39 | $CH_3$ | $CH_3$ | H | $C_2H_5$ | H | |
| 40 | $CH_3$ | H | H | H | H | |
| 41 | $CH_3$ | $CH_3$ | 3-Cl | H | H | |
| 42 | i-$C_3H_7$ | H | H | H | H | |
| 43 | $CH_3$ | $C_2H_5$ | H | H | $COOCH_3$ | 105 |
| 44 | $CH_3$ | $C_2H_5$ | H | H | Cl | |
| 45 | $CH_3$ | $C_2H_5$ | H | H | H | 90 |
| 46 | $CH_3$ | $C_2H_5$ | H | $C_2H_5$ | $COOC_2H_5$ | |
| 47 | $CH_3$ | $C_2H_5$ | H | $C_2H_5$ | H | |
| 48 | $CH_3$ | Cl | 4-Cl | H | H | 127 |
| 49 | $C_2H_5$ | $C_2H_5$ | H | H | $COOCH_3$ | 59 |
| 50 | $C_2H_5$ | $C_2H_5$ | H | H | Cl | |
| 51 | $C_2H_5$ | $C_2H_5$ | H | H | H | 99-102 |
| 52 | i-$C_3H_7$ | i-$C_3H_7$ | H | H | H | |
| 53 | $CH_3$ | $CH_2$-CH=$CH_2$ | H | H | $COOCH_2CH(CH_3)_2$ | |
| 54 | $CH_3$ | $CH_2$-CH=$CH_2$ | H | H | H | |
| 55 | $CH_3$ | $CH_3$ | 3-$CH_3$ | H | $COOCH_3$ | |

BAD ORIGINAL

0086477



| Nr. | $R_1$ | $R_2$ | $R_3$ | $R_4$ | $R_5$ | Schmp. (°C) |
|---|---|---|---|---|---|---|
| 56 | $CH_3$ | Cl | H | $CH_3$ | $COOC_3H_7(n)$ | |
| 57 | $CH_3$ | Cl | H | $CH_3$ | H | |
| 58 | $CH_3$ | $CH_3$ | H | $COOCH_3$ | H | |
| 59 | $CH_3$ | $CH_3$ | H | $COOC_2H_5$ | $C_2H_5$ | |
| 60 | $CH_3$ | $CH_3$ | H | $CH_3$ | H | |
| 61 | $CH_3$ | $C_2H_5$ | H | $CH_3$ | H | |
| 62 | $C_2H_5$ | $C_2H_5$ | H | $CH_3$ | H | |
| 63 | $CH_3$ | $CH_3$ | H | H | $CH_3$ | |
| 64 | $CH_3$ | $C_2H_5$ | H | $CH_3$ | $CH_3$ | |
| 65 | $C_2H_5$ | $C_2H_5$ | H | $CH_3$ | $CH_3$ | |
| 66 | $CH_3$ | $CH_3$ | H | $-CH_2-CH_2-$ | | |
| 67 | $CH_3$ | $CH_3$ | H | $-CH_2-CH_2-CH_2-$ | | |
| 68 | $CH_3$ | $C_2H_5$ | H | H | COOH | |

BAD ORIGINAL

## FORMULIERUNGSBEISPIELE

### Beispiel A

Ein emulgierbares Konzentrat wird erhalten aus

| | | |
|---|---|---|
| | 15 Gew.-Teilen | Wirkstoff |
| | 75 Gew.-Teilen | Cyclohexanon als Lösungsmittel |
| und | 10 Gew.-Teilen | oxäthyliertes Nonylphenol (10 AeO) als Emulgator. |

### Beispiel B

Ein in Wasser leicht dispergierbares benetzbares Pulver wird erhalten, indem man

| | | |
|---|---|---|
| | 25 Gew.-Teile | Wirkstoff |
| | 64 Gew.-Teile | kaolinhaltiges Quarz als Inertstoff |
| | 10 Gew.-Teile | ligninsulfonsaures Kalium |
| und | 1 Gew.-Teile | oleylmethyltaurinsaures Natrium als Netz- und Dispergiermittel |

mischt und in einer Stiftmühle mahlt.

### Beispiel C

Ein Stäubemittel wird erhalten, indem man

| | | |
|---|---|---|
| | 10 Gew.-Teile | Wirkstoff |
| und | 90 Gew.-Teile | Talkum als Inertstoff |

mischt und in einer Schlagmühle zerkleinert.

BAD ORIGINAL

Beispiel D

Ein Granulat besteht z.B. aus etwa

2 bis 15 Gew.-Teilen Wirkstoff
98 bis 85 Gew.-Teilen inerten Granulatmaterialien, wie z.B. Attapulgit, Bimstein und Quarzsand.

BAD ORIGINAL

## BIOLOGISCHE BEISPIELE

### 1. Herbizide Vorauflaufwirkung

Samen bzw. Rhizomstücke annueller und perennierender Unkräuter werden in Plastiktöpfen (Ø 9 cm) in sandigem Lehmboden ausgesät und mit einem leichten Sandboden abgedeckt. Die als benetzbare Pulver formulierten erfindungsgemäßen Verbindungen wurden in Form wässriger Suspensionen oder Emulsionen auf die Erdoberfläche appliziert. Die Wasseraufwandmenge pro Topf entsprach dabei umgerechnet 600 l/ha. Nach der Behandlung wurden die Versuchstöpfe im Gewächshaus aufgestellt und die Versuchspflanzen unter guten Wachstumsbedingungen (Temperatur 23 $\pm$ 1°C; rel. Luftfeuchte 60 - 80 %) kultiviert. Nach ca. 3 Wochen wurde die Pflanzenschädigung im Vergleich zur unbehandelten Kontrolle optisch bonitiert. In Tabelle 1 ist die Vorauflaufwirksamkeit der neuen Verbindungen zusammengestellt. Die angegebenen Zahlenwerte zeigen klar, daß die erfindungsgemäßen Verbindungen eine gute herbizide Wirkung sowohl gegen monokotyle als auch gegen dikotyle Schadpflanzen aufweisen, wenn die Wirkstoffe im Vorauflaufverfahren appliziert werden.

BAD ORIGINAL

<u>Tabelle 1</u>

Vorauflaufwirkung gegen mono- und dikotyle Unkräuter

| Verbindung (Beisp.-Nr.) | Dosis kg a.i./ha | herbizide Wirkung gegen ALM | SAL | CYI | AMR | CYE |
|---|---|---|---|---|---|---|
| 33 | 2,4 | 5 | 5 | 5 | 5 | 5 |
| | 0,6 | 5 | 5 | 5 | 5 | - |
| | 0,15 | 5 | 5 | 5 | 2 | - |
| 34 | 2,4 | 3 | 5 | 5 | 5 | 0 |
| 35 | 2,4 | 5 | 5 | 5 | 5 | 5 |
| | 0,6 | 3 | 5 | 5 | 5 | - |
| | 0,15 | 0 | 5 | 5 | 3 | - |
| 43 | 2,4 | 1 | 4 | 5 | 5 | 0 |
| 45 | 2,4 | 5 | 5 | 5 | 5 | 5 |
| | 0,6 | 3 | 5 | 5 | 5 | - |
| | 0,15 | 2 | 5 | 5 | 5 | - |
| 49 | 2,4 | 0 | 4 | 5 | 4 | 0 |
| 51 | 2,4 | 5 | 5 | 5 | 5 | 5 |
| | 0,6 | 3 | 5 | 5 | 5 | - |
| | 0,15 | 2 | 3 | 5 | 3 | - |

Dabei bedeutet

0= ohne Wirkung
1= < 20 % Wirkung
2= 20 - 40 % Wirkung
3= 40 - 60 % Wirkung
4= 60 - 80 % Wirkung
5= 80 -100 % Wirkung
-= nicht geprüft

ALM= Alopecurus myosuroides
SAL= Setaria lutescens
CYI= Cyperus iria
AMR= Amaranthus retroflexus
CYE= Cyperus esculentus
a.i.= Aktivsubstanz

PATENTANSPRÜCHE:

1. Verbindungen der allgemeinen Formel I

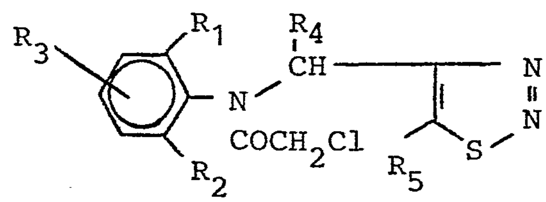

(I)

worin

$R_1$ $(C_1-C_3)$Alkyl,
$R_2$ (Wasserstoff, $C_1-C_3$)Alkyl, $(C_3-C_5)$Alkenyl, $(C_1-C_3)$Alkoxymethyl, Halogen oder $(C_1-C_3)$Alkoxy
$R_3$ Wasserstoff, Chlor, Brom oder Methyl,
$R_4$ Wasserstoff, $(C_1-C_3)$Alkyl oder COO-$(C_1-C_4)$Alkyl
$R_5$ Wasserstoff, $(C_1-C_3)$Alkyl, Halogen, Carboxy oder $(C_1-C_4)$Alkoxycarbonyl

oder $R_4$ und $R_5$ zusammen Trimethylen oder Ethylen

bedeuten, sowie, falls $R_5$= COOH, deren Salze mit Basen.

2. Verbindungen der Formel I gemäß Anspruch 1, dadurch gekennzeichnet, daß $R_1$ und $R_2$ $(C_1-C_3)$Alkyl, $R_3$ und $R_4$ Wasserstoff und $R_5$ Wasserstoff, Halogen oder $(C_1-C_4)$-Alkoxycarbonyl bedeuten.

3. Verbindungen der Formel I gemäß Anspruch 1, dadurch gekennzeichnet, daß $R_1$ und $R_2$ Methyl, $R_3$ und $R_4$ Wasserstoff und $R_5$ Wasserstoff, Chlor oder Methoxycarbonyl bedeuten.

4. Chloressigsäure-N-(1,2,3-thiadiazol-4-ylmethyl)-2,6-dimethylanilid.

5. Chloressigsäure-N-(5-chlor-1,2,3-thiadiazol-4-ylmethyl)-2,6-dimethylanilid.

BAD ORIGINAL

0086477
HOE 82/F 027

6. Chloressigsäure-N-(1,2,3-thiadiazol-3-yl-methyl)-2-ethyl-6-methylanilid.

7. Verfahren zur Herstellung von Verbindungen der Formel I, dadurch gekennzeichnet, daß man Amine der Formel II

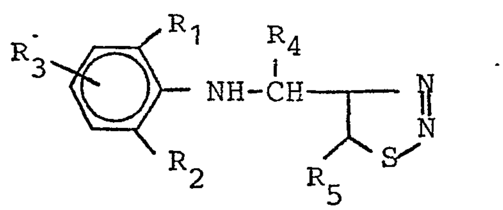

(II)

mit Chloracetylierungsmitteln behandelt.

8. Mittel zur Bekämpfung von unerwünschtem Pflanzenwuchs, gekennzeichnet durch einen Gehalt an einer Verbindung der Formel I.

9. Verwendung von Verbindungen der Formel I zur Bekämpfung unerwünschten Pflanzenwuchses.

10. Verfahren zur Bekämpfung unerwünschten Pflanzenwuchses, dadurch gekennzeichnet, daß man auf die zu behandelnden Flächen eine wirksame Menge einer Verbindung der Formel I aufbringt.

BAD ORIGINAL

0086477
HOE 82/F 027

## Patentansprüche für Österreich:

1. Verfahren zur Herstellung von Verbindungen der Formel I

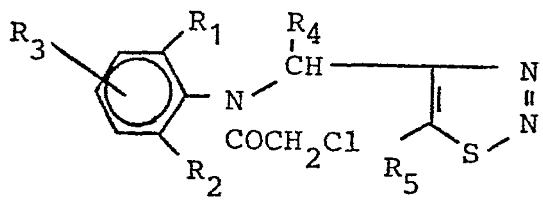

(I)

worin

$R_1$ $(C_1-C_3)$Alkyl,

$R_2$ (Wasserstoff, $C_1-C_3$)Alkyl, $(C_3-C_5)$Alkenyl, $(C_1-C_3)$Alkoxymethyl, Halogen oder $(C_1-C_3)$Alkoxy

$R_3$ Wasserstoff, Chlor, Brom oder Methyl,

$R_4$ Wasserstoff, $(C_1-C_3)$Alkyl oder COO-$(C_1-C_4)$Alkyl

$R_5$ Wasserstoff, $(C_1-C_3)$Alkyl, Halogen, Carboxy oder $(C_1-C_4)$Alkoxycarbonyl

oder $R_4$ und $R_5$ zusammen Trimethylen oder Ethylen

bedeuten, sowie, falls $R_5$= COOH, von deren Salzen mit Basen, dadurch gekennzeichnet, daß man Amine der Formel II

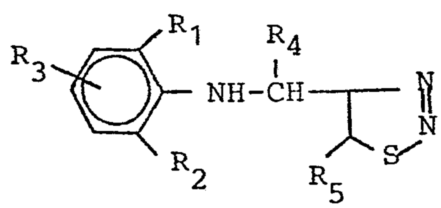

(II)

mit Chloracetylierungsmitteln behandelt.

2. Mittel zur Bekämpfung von unerwünschtem Pflanzenwuchs, gekennzeichnet durch einen Gehalt an einer Verbindung der Formel I.

3. Verwendung von Verbindungen der Formel I zur Bekämpfung unerwünschten Pflanzenwuchses.

BAD ORIGINAL

4. Verfahren zur Bekämpfung unerwünschten Pflanzenwuchses, dadurch gekennzeichnet, daß man auf die zu behandelnden Flächen eine wirksame Menge einer Verbindung der Formel I aufbringt.

BAD ORIGINAL

Europäisches Patentamt

# EUROPÄISCHER RECHERCHENBERICHT

0086477
Nummer der Anmeldung

EP 83 10 1347

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| P | EP-A-0 046 497 (BASF)<br>* Ansprüche; Seiten 25-27 *<br>----- | 1,7-10 | C 07 D 285/06<br>A 01 N 43/82 |
| | | | **RECHERCHIERTE SACHGEBIETE (Int. Cl. 3)** |
| | | | C 07 D 285/00 |

Der vorliegende Recherchenbericht wurde für alle Patentansprüche erstellt.

| Recherchenort | Abschlußdatum der Recherche | Prüfer |
|---|---|---|
| DEN HAAG | 26-05-1983 | CREMERS K. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82